Europäisches Patentamt

European Patent Office

Office européen des brevets

(11) Veröffentlichungsnummer: **0 405 569 A1**

(12) **EUROPÄISCHE PATENTANMELDUNG**

(21) Anmeldenummer: 90112401.6

(22) Anmeldetag: 29.06.90

(51) Int. Cl.5: **C12P 1/04, A61K 35/66,**
//(C12P1/04,C12R1:46)

(30) Priorität: 30.06.89 CH 2433/89

(43) Veröffentlichungstag der Anmeldung:
02.01.91 Patentblatt 91/01

(84) Benannte Vertragsstaaten:
AT BE CH DE DK ES FR GB IT LI LU NL SE

(71) Anmelder: **Cernitin S.A.**

**CH-6911 Barbengo(CH)**

(72) Erfinder: **Lewenstein, Ari, Dr.**
**Via Marco da Carona 15**
**CH-6900 Lugano(CH)**
Erfinder: **Crivelli, Paolo, Dr.**
**Via Guidino 41**
**CH-6900 Lugano/Paradiso(CH)**
Erfinder: **Jaton, Jean-Claude, Prof.**
**Ave. Adrien Jeandin 14**
**CH-1226 Thonex(CH)**

(74) Vertreter: **Blum, Rudolf Emil Ernst et al**
**c/o E. Blum & Co Patentanwälte Vorderberg**
**11 11**
**CH-8044 Zürich(CH)**

(54) **Produkt mit antimikrobieller Wirksamkeit, Verfahren zu dessen Isolierung aus dem Kulturmedium von Streptokokkus faecium und pharmazeutisches Präparat, enthaltend dieses Produkt mit antimikrobieller Wirksamkeit.**

(57) Ein Produkt, das aus dem wässrigen Kulturmedium von Streptokokkus faecium isoliert wird, weist eine wachstumshemmende Wirksamkeit gegenüber Mikroorganismen auf, insbesondere sowohl gegenüber grampositiven als auch gegenüber gramnegativen Bakterien. Der Wirkstoff besitzt ein Molekulargewicht von weniger als 1000 Dalton, er verliert bei einer zwanzigminütigen Erhitzung auf 80° C seine Wirksamkeit nicht und ist in einem wässrigen Medium eines etwa neutralen pH-Wertes im Bereich von 6,0 - 7,5 löslich, und aus diesem durch Chloroform nicht extrahierbar.

Der antimikrobielle Wirkstoff, beziehungsweise das entsprechende Wirkstoffgemisch ist in pharmazeutischen Präparaten zur Bekämpfung von Infektionen verwendbar, insbesondere oral zu verabreichenden Präparaten zur Bekämpfung von Störungen im Magen-Darm-Trakt.

EP 0 405 569 A1

# PRODUKT MIT ANTIMIKROBIELLER WIRKSAMKEIT, VERFAHREN ZU DESSEN ISOLIERUNG AUS DEM KULTURMEDIUM VON STREPTOKOKKUS FAECIUM UND PHARMAZEUTISCHES PRÄPARAT, ENTHALTEND DIESES PRODUKT MIT ANTIMIKROBIELLER WIRKSAMKEIT.

## HINTERGRUND DER ERFINDUNG

Pharmazeutische Präparate, die lebende Mikroorganismen enthalten, sind seit vielen Jahren bekannt und entsprechende pharmazeutische Präparate, die als lebende Mikroorganismen Streptokokkus faecium enthalten, werden seit mehreren Jahren oral verabreicht um Störungen im Magen-Darm-Trakt zu bekämpfen. Pharmazeutische Präparate, die lebende Mikroorganismenstämme enthalten, weisen jedoch eine begrenzte Haltbarkeit auf und ausserdem kann der Verbraucher nicht feststellen, ob in dem Präparat noch eine ausreichend hohe Anzahl an tatsächlich lebenden Mikroorganismen enthalten ist, damit dieses seine Wirksamkeit entfalten kann.

Ziel der vorliegenden Erfindung war es daher, aus Streptokokkus faecium ein Produkt zu isolieren, das mindestens einen Wirkstoff mit antimikrobieller Wirksamkeit enthält oder daraus besteht, wobei dieses Produkt als aktiver Bestandteil von pharmazeutischen Präparaten einsetzbar sein soll, welche nicht diejenigen Nachteile aufweisen, die bei pharmazeutischen Präparaten anzutreffen sind, welche entsprechende lebende Mikroorganismen enthalten.

## BESCHREIBUNG DES STANDES DER TECHNIK

Es ist bekannt, dass häufig bei der Züchtung von Bakterienstämmen Produkte an das wässrige Kulturmedium abgegeben werden, welche gegenüber solchen Bakterienstämmen, die mit den gezüchteten Bakterienstämmen nahe verwandt sind, bakterizide Eigenschaften aufweisen. Diese Produkte werden "Bakteriozine" genannt. Sie besitzen im allgemeinen keinerlei Wirksamkeit gegenüber solchen Mikroorganismen, welche mit den gezüchteten Bakterienstämmen nicht nahe verwandt sind. So besitzen beispielsweise Bakteriozine, die von Milchsäurebakterien, beispielsweise Lactobazillen oder Streptokokken, in das Nährmedium ausgeschieden werden, üblicherweise keine Wirksamkeit gegenüber krankheitserregenden Mikroorganismen aus der Gruppe Coli oder Salmonella.

Es ist ferner bekannt, in der Humanmedizin und in der Veterinärmedizin pharmazeutische Präparate oral zu verabreichen, die lebende Mikroorganismen enthalten, welche in der ungestörten Darmflora von Säugetieren, beziehungsweise Menschen enthalten sind, um Störungen im Magen-Darmtrakt zu bekämpfen, beispielsweise nach einer Verabreichung von die Darmflora abtötenden Antibiotika, beziehungsweise um Verdauungsstörungen, insbesondere Durchfälle, zu bekämpfen. Diese lebende Mikroorganismen enthaltenden Präparate enthalten entweder nur einen Mikroorganismenstamm oder mehrere unterschiedliche Mikroorganismenstämme, zum Beispiel nicht pathogene Stämme aus der Gruppe Lactobacillus oder Streptokokkus, wie zum Beispiel Streptokokkus faecium, beispielsweise den Stamm Streptokokkus faecium Cernelle 68 mit der Bezeichnung SF68.

Mit derartigen lebende Mikroorganismen stämme enthaltenden pharmazeutischen Präparaten werden im allgemeinen gute Erfolge bei einer prophylaktischen Behandlung zur Verhinderung einer Infektion mit pathogenen Mikroorganismenstämmen erzielt und auch bei der Behandlung von bereits aufgetretenen Magen-Darmstörungen, beispielsweise heftigen Durchfällen, sind die Erfolge in manchen Fällen gut.

Ein Nachteil dieser lebende Mikroorganismen enthaltenden Präparate besteht jedoch darin, dass nach einiger Lagerungszeit allmählich die Zahl der lebenden Mikroorganismen abnimmt, insbesondere wenn die Präparate nicht im Kühlschrank, sondern bei Umgebungstemperaturen gelagert werden, und für den Verbraucher ist es ferner aus dem Zustand der Präparate nicht ersichtlich, ob die Anzahl der darin enthaltenen tatsächlich lebenden Mikroorganismen noch ausreichend hoch zur Erzielung der entsprechenden Wirksamkeit ist.

Ein weiterer Nachteil dieser lebende Mikroorganismen enthaltenden Präparate besteht darin, dass die Vermehrung der Mikroorganismen nach der oralen Verabreichung dieser Produkte sehr stark von den im Magen-Darm-Trakt vorherrschenden Bedingungen abhängt. Bei schlechtem Wachstum der verabreichten lebenden Mikroorganismen ist natürlich die therapeutische Wirkung gering. Besonders nachteilig ist es jedoch, dass nicht vorhergesehen werden kann, ob nach der Verabreichung der lebenden Mikroorganismen deren Vermehrung im Magen-Darm-Trakt gut oder weniger gut ist.

Bisher wurden ferner die von Mikroorganismenstämmen in das Kulturmedium abgegebenen Wirkstoffe nicht aus dem Kulturmedium isoliert und als solche verabreicht, sondern es erfolgte immer die Verabreichung der lebende Mikroorganismen enthaltenden Präparate.

In der Veröffentlichung von Kawai, Yasuo und Ishihara, Kazuoki in Chemical Abstracts, Band 105, 1986, Seite 568, Zusammenfassung Nr. 151550s wird eine Substanz mit anitmikrobieller Wirksamkeit beschrieben, welche als PKI bezeichnet wird, und welche die folgende Summenformel

$C_{11}H_{15}N_5O_5$

aufweist. Zur Gewinnung dieser antimikrobiell wirkenden Substanz wurde der Mikroorganismusstamm Steptokokkus faecium FFRM P 7538 in einem wässrigen Kulturmedium gezüchtet, man isolierte dann die Mikroorganismenzellen aus dem Kulturmedium und wusch sie, um anhaftende Spuren des Kulturmediums zu entfernen und zerstörte schliesslich die Zellen durch eine Behandlung im Autoklaven bei einer Temperatur von 110 - 121° C. Aus diesem Produkt wurden dann die Zellhüllen durch Zentrifugieren abgetrennt und die überstehende Flüssigkeit mit Aethylacetat oder Butanol extrahiert. Der entsprechende Wirkstoff wurde aus der organischen Phase isoliert und zur Eindämmung einer unerwünschten Mikroorganismenflora in der Mundhöhle eingesetzt, beispielsweise als Komponente von Zahnpasten, Mundspühlmitteln und Kaugummis.

Der in diesem Abstract beschriebene Wirkstoff der angeführten Summenformel wird also nicht von den lebenden Mikroorganismen an das wässrige Kulturmedium abgegeben, sondern der fragliche Wirkstoff wird nach der Zerstörung der Mikroorganismenzellen aus dem Zellinhalt gewonnen.

In der Veröffentlichung Chemical Abstracts, Band 105, 1986, Seite 353, Zusammenfassung Nr. 11878e von Kawai, Yasuo und Ishihara, Kazuoki wird erwähnt, dass die Substanz mit der Bezeichnung PKI, welche die oben angegebene Summenformel aufweist, als antimikrobieller Bestandteil von Zahnpflegemitteln, Mundspühlmitteln und Kaugummis eingesetzt werden kann.

In der US Patentschrift 4,210,673 von Endre Kvanta, wird ein Verfahren zur Fermentierung von Grünfutter beschrieben, bei dem man dem Grünfutter eine Kultur von Streptokokkus faecium zusetzt, die ein Stoffwechsel produkt bildet, welches eine Wirksamkeit gegenüber Escherichia coli (Anti-Escherichia-coli-Aktivität) aufweist. Die Vergärung oder Fermentation des Grünfutters wird in Anwesenheit von Stickstoff liefernden Materialien, abbaubaren Kohlenhydraten und essentiellen Wachstumsfaktoren bei einer Temperatur von mindestens 15° C durchgeführt, bis die im gepressten Grünfutter gebildete Menge an Milchsäure mindestens 2% erreicht hat. In Spalte 3, Zeile 61, bis Spalte 4, Zeile 4, dieser US Patentschrift wird ferner klargelegt, dass im Gegensatz zu verschiedenen anderen milchsäurebildenden Stämmen von Streptokokkus und Lactobacillus das zellfreie Medium von Kulturen von Streptokokkus faecium eine starke Anti-Escherichia-coli-Aktivität aufweist. Es wird angenommen, dass dieser Effekt entweder durch eine Substanz mit bakteriostatisch/bakteriozider Wirksamkeit, wie 2-Desoxi-D-glucose, oder durch eine Substanz hervorgerufen wird, die als Aktivator des sogenannten Lactoperoxidase-systems dient, wie zum Beispiel Wasserstoffperoxid. Offensichtlich ist diese Substanz mit bakteriostatischer, beziehungsweise bakteriozider Wirksamkeit oder der Aktivator in dem Kulturmedium anwesend. In dieser US Patentschrift werden jedoch keinerlei Verfahrensschritte erwähnt, die zur Isolierung der entsprechenden Substanz aus dem Kulturmedium dienen könnten und nur das entsprechende vergorene Grünfutter, welches die noch lebenden Mikroorganismen enthält, wird nach dem dort beschriebenen Verfahren hergestellt und an die Tiere verfüttert.

In der französischen Patentschrift 2,550,223 von S. Udaka et al. wird eine Substanz beschrieben, die von einem Streptokokkus gewonnen wird, der als SPF-100 bezeichnet wird, und die eine tumorhemmende Aktivität besitzt. Auf Seite 22, Punkt 7, dieser Patentschrift wird klargelegt, dass diese tumorizide Substanz in Wasser, Methanol und Aethanol löslich ist, jedoch völlig unlöslich in höheren Alkoholen, niederen Ketonen und anderen organi nischen Lösungsmitteln, wie n-Propanol, n-Butanol, Iso-propanol, Aceton, Methyl-isobutyl-keton, Diäthyläther, n-Hexan und Chloroform. Auf Seite 22, Punkt 5, dieser französischen Patentschrift wird ferner erwähnt, dass diese tumorizide Substanz ein Molekulargewicht im Bereich von 500 - 25 000 (entsprechend der Bestimmung durch Gelfiltration) aufweist und dass eine 0,1%-ige wässrige Lösung dieser Substanz im Ultraviolettspektrum Absorptionen bei 257, 265, 280 und 325 nm zeigt.

In der Veröffentlichung von R.C. Moellering et al., Chemical Abstracts, Band 91, 1979, Seiten 61-62, Zusammenfassung Nr. 186755g, werden Kombinationen des Antibiotikums Penicillin G mit verschiedenen aminoglycosidischen Aminocyclitolen bezüglich ihrer in vitro Aktivität gegenüber einer Anzahl von klinisch isolierten Stämmen von Streptokokkus faecium und Streptokokkus faecalis getestet. Dabei zeigte es sich, dass Streptokokkus faecium gegenüber diesem Antibiotikum eine höhere Resistenz aufweist als Streptokokkus faecalis.

Die Empfindlichkeit von klinisch isolierten Stämmen von Streptokokkus faecium and Streptokokkus faecalis gegenüber verschiedenen antimikrobiellen Wirkstoffen wurde auch von M.J. Kim et al. getestet und es sei in diesem Zusammenhang auf Chemical Abstracts, Band 107, 1987, Seite 382, Zusammenfassung Nr. 4130t verwiesen.

In der Veröffentlichung von L.D. Liebowitz et al. aus der Zeitschrift Antimicrobial Agents and Chemotherapy, Band 32, Nr. 1, Januar 1988, Seiten 24-26 der American Society for Microbiology werden klinisch isolierte Bakterienstämme untersucht und es wird ihre Resistenz gegen ein neues zyklisches Lipopeptid mit der Bezeichnung LY146032 getestet.

Bereits im Jahre 1979 haben die Autoren A. Lewenstein, G. Frigerio und M. Moroni festgestellt, dass ein milchsäureproduzierender Stamm von Streptokokkus faecium, nämlich derjenige Stamm, der bei der NCIB als SF68 hinterlegt ist, in Mischkulturen dieses Streptokokkus faecium Stammes mit Escherichia coli und in Mischkulturen dieses Mikroorganismenstammes mit Salmonella typhimurium eine wachtumshemmende Aktivität gegenüber diesen pathogenen Mikroorganismenstämmen aufweist. Es sei in diesem Zusammenhang auf die entsprechende Veröffentlichung dieser Autoren in Current Therapeutic Research, Band 26, Nr. 6, Abschnitt 2, Dezember 1979, Seiten 967-981 verwiesen.

Trotz der Tatsache, dass seit langem pharmazeutische Präparate, die lebende Mikroorganismenstämme von Streptokokkus faecium enthalten und solche pharmazeutischen Präparate, die lebende Mikroorganismen des hinterlegten Streptokokkus faecium Stammes mit der Bezeichnung SF68 enthalten, zur Behandlung von Störungen im Magen-Darm-Trakt eingesetzt wurden, sind bisher noch keine antimikrobiell wirksamen Bestandteile aus Streptokokkus faecium selbst oder aus dem Kulturmedium, in welchem dieser gezüchtet wurde, isoliert worden, die zu den angegebenen Zwecken einsetzbar sind.

Ziel der vorliegenden Erfindung war es, ein neues Produkt mit antimikrobieller Wirksamkeit zur Verfügung zu stellen, welches diejenigen Nachteile nicht besitzt, welche solchen Produkten anhaften, die lebende Mikroorganismen enthalten. Insbesondere sollte das herzustellende von lebenden Mikroorganismen freie Produkt eine bessere Lagerfähigkeit aufweisen als lebende Mikroorganismen enthaltende Produkte und es sollte ferner bei der oralen Verabreichung eine konstante und vorhersehbare Wirksamkeit besitzen.

Ein weiteres Ziel der vorliegenden Erfindung war es, ein Produkt mit antimikrobieller Wirksamkeit zur Verfügung zu stellen, welches das Wachstum einer Vielzahl von miteinander nicht nahe verwandten pathogenen Mikroorganismenstämmen hemmt.

Ueberraschenderweise zeigte es sich, dass aus dem wässrigen Kulturmedium von Streptokokkus faecium isolierte Produkte die angestrebten Eigenschaften einer recht breiten antimikrobiellen Wirksamkeit und einer guten Lagerfähigkeit besitzen.

## BESCHREIBUNG DER ERFINGUNG

Ueberraschenderweise zeigte es sich, dass aus dem wässrigen Kulturmedium von Streptokokkus faecium, nach der Entfernung der Mikroorganismen, ein Produkt isoliert werden kann, das ein Molekulargewicht unterhalb von 1000 Dalton aufweist und das seine Wirksamkeit nicht verliert, wenn es in einem wässrigen Medium mit einem einen etwa neutralen pH-Wert mit einem Peptidbindungen spaltenden Enzym behandelt wird, das ferner eine gute Wärmebeständigkeit aufweist und das aus einem neutralen bis schwach alkalischen wässrigen Medium mit einem wasserunmischbaren Lösungsmittel nicht extrahierbar ist.

Ein Gegenstand der vorliegenden Erfindung ist daher ein Produkt mit antimikrobieller Wirksamkeit, das dadurch gekennzeichnet ist, dass es aus dem Kulturmedium von Streptokokkus faecium isoliert ist und dass dieses Produkt mindestens einen Wirkstoff enthält oder aus mindestens einem Wirkstoff besteht, der ein Molekulargewicht unter 1000 Dalton aufweist und die folgenden Eigenschaften besitzt:

(a) der mindestens eine Wirkstoff verliert bei einem pH-Wert im Bereich von 6,2 - 7,5 bei einer Behandlung mit einem Peptidbindungen spaltenden Enzym seine Wirksamkeit nicht;

(b) der mindestens eine Wirkstoff verliert bei einer dreissigminütigen Erhitzung auf 60° und/oder zwanzigminütigen Erhitzung auf 80° seine Wirksamkeit nicht und

(c) der mindestens eine Wirkstoff ist in einem wässrigen Medium eines etwa neutralen bis alkalischen pH-Wertes im Bereich von 6,0 - 9,0 löslich und er ist aus dem wässrigen Medium dieses etwa neutralen bis schwach alkalischen pH-Wertes mit einem wasserunmischbaren organischen Lösungsmittel nicht extrahierbar.

Es werden derzeit intensive Forschungen durchgeführt, um die chemische Struktur des Wirkstoffes und/oder allenfalls eines Wirkstoffgemisches mit einem Molekulargewicht von jeweils unter 1000 Dalton genauer aufzuklären. Sicher ist einstweilen, dass der fragliche Wirkstoff keine solche Säureamidgruppierung enthält, die von Enzymen, welche Peptidbindungen spalten, hydrolytisch aufgespalten wird. Es zeigte sich, dass ein den Wirkstoff, beziehungsweise allenfalls ein Wirkstoffgemisch enthaltendes Produkt in einem wässrigen Medium bei einem pH-Wert von 6,2 - 7,5 seine Wirksamkeit behält, wenn in diesem wässrigen Medium solche Peptidbindungen spaltenden Enzyme anwesend sind, die aus der Gruppe ausgewählt sind, welche Desoxyribonuklease, Ribonukleas, Trypsin und Pronase umfasst.

Der in dem erfindungsgemässen Produkt enthaltene Wirkstoff, beziehungsweise das Wirkstoffgemisch mit antimikrobieller Wirksamkeit ist in wässrigen Medien eines pH-Wertes von 6,0 - 9,0 , beispielsweise eines pH-Wertes im Bereich von 6,9 - 7,5 , gut löslich.

Wenn ferner das wässrige Medium, dessen pH-Wert im Bereich von 6,0 - 9,0 liegt, mit einem mit Wasser nicht mischbaren organischen Lösungsmittel extrahiert wird, dann bleibt der Wirkstoff in dem wässrigen Medium zurück und geht nicht in die organische Phase über, und wenn die abgetrennte Schicht des organischen Lösungsmittels abgetrennt wird, dann zeigt der verbleibende Rückstand entweder überhaupt keine antimikrobielle Aktivität oder nur eine überaus geringe, praktisch vernachlässigbare antimikrobielle Aktivität. Durch diese Extraktion mit den wasserunmischbaren organischen Lösungsmitteln werden also aus der einen pH-Wert von 6,0 - 9,0 , beispielsweise einen pH-Wert von 6,0 - 7,5 aufweisenden wässrigen Phase nur nicht aktive Nebenprodukte niedriger Polarität entfernt.

Bevorzugte Beispiele für wasserunmischbare organische Lösungsmittel, mit denen aus einem wässrigen Medium eines pH-Wertes von 6,0 - 9,0 der Wirkstoff nicht entfernt wird, sind Kohlenwasserstoffe und halogeniert Kohlenwasserstoffe, vorzugsweise chlorierte Kohlenwasserstoffe, und als spezielle Beispiele seien aliphatische und aromatische Kohlenwasserstoffe, wie Erdölfraktionen, beispielsweise niedrig siedende Erdölfraktionen, wie Petroläther, Benzol, Toluol und chlorierte Kohlenwasserstoffe, wie Chloroform oder Tetrachlokohlenstoff genannt. ·

Wird jedoch der pH-Wert des Wirkstoff enthaltenden wässrigen Mediums auf einen sauren pH-Wert von 3,0 oder unterhalb von 3,0 gesenkt, beispielsweise auf einen pH-Wert im Bereich von 1,5 - 2,5 , dann ist der mindestens eine Wirkstoff aus dem wässrigen Medium mit einem wasserunmischbaren organischen Lösungsmittel extrahierbar.

Der pH-Wert des wässrigen Mediums wird zweckmässigerweise durch Zugabe einer Säure, insbesondere einer anorganischen Säure, wie Chlorwasserstoffsäure, auf den sauren pH-Wert von 3,0 oder unterhalb von 3,0 , eingestellt und dann der Wirkstoff aus diesem sauren wässrigen Medium mit einem wasserunmischbaren organischen Lösungsmittel extrahiert. Es zeigte sich, dass nach der Abtrennung der organischen Phase und dem Eindampfen des organischen Lösungsmittels ein Rückstand verbleibt, der eine sehr hohe antimikrobielle Wirksamkeit besitzt. Die saure wässrige Phase, die nach der Extraktion mit dem wasserunmischbaren Lösungsmittel zurückbleibt, wurde dann durch Zugabe einer Base, vorzugsweise einer anorganischen Base, auf einen etwa neutralen pH-Wert, eingestellt und das Wasser unter Vakuum abgedampft. Es zeigte sich, dass der dabei verbleibende Rückstand keine antimikrobielle Aktivität oder praktisch keine antimikrobielle Aktivität, aufweist.

Die organischen Lösungsmittel, die zur Entfernung des Wirkstoffes oder Wirkstoffgemisches aus der auf einen pH-Wert von 3,0 oder unterhalb von 3,0 eingestellten wässrigen Lösung eingesetzt werden, können die gleichen organischen Lösungsmittel sein, die bereits weiter oben für die Entfernung der nicht wirksamen unpolaren Bestandteile aus dem einen neutralen bis alkalischen pH-Wert aufweisenden wässrigen Medium genannt wurden. Es sind also zur Extraktion der Wirkstoffe aus dem sauren wässrigen Medium Kohlenwasserstoffe, wie zum Beispiel aliphatische, alicyklische oder aromatische Kohlenwasserstoffe, sowie halogenierte Kohlenwasserstoffe, geeignet. Vorzugsweise verwendet man jedoch zur Extraktion des Wirkstoffes, beziehungsweise Wirkstoffgemisches aus dem einen sauren pH-Wert aufweisenden wässrigen Medium ein wasserunmischbares Lösungsmittel, das eine etwas höhere Polarität aufweist als Kohlenwasserstoffe, insbesondere einen Dialkyläther oder einen cyklischen wasserunmischbaren Aether, wie zum Beispiel Diäthyläther oder einen Ester.

Die erfindungsgemässen Produkte, beziehungsweise der in ihnen enthaltene Wirkstoff, und/oder das in ihnen enthaltene Wirkstoffgemisch, weist eine wachstumshemmende Wirksamkeit gegen eine grosse Anzahl an Mikroorganismenstämmen auf, die mit dem Stamm von Streptokokkus faecium und/oder den Stämmen von Streptokokkus faecium des Kulturmediums, aus dem der Wirkstoff, beziehungsweise das Wirkstoffgemisch isoliert wurde, nicht nahe verwandt sind. Die erfindungsgemässen Produkte, beziehungsweise die .in ihnen enthaltenen Wirkstoffe, weisen eine wachstumshemmende Wirksamkeit gegenüber grampositiven und gramnegativen Stämmen auf, zum Beispiel Stämmen von Streptokokkus faecalis, Escherichia coli, Salmonella, Staphylococcus aureus, Shigella, Bacillus subtilis und/oder Listeria.

Es zeigte sich jedoch, dass die entsprechenden Produkte, bzw. die in ihnen enthaltenden Wirkstoffe oder Wirkstoffgemische im allgemeinen keine wachstumshemmende Wirkung oder nur eine geringe wachstumshemmende Wirkung gegenüber Mikroorganismenstämmen der Familie Klebsiella aufweisen.

Die antimikrobielle Wirksamkeit der erfindungsgemässen Produkte konnte in vitro anhand der Hemmung des Wachstums entsprechender Mikroorganismenkulturen nachgewiesen werden und ferner auch bei entsprechenden in vivo Versuchen.

Zu diesem Zwecke wurden gontobiotische Tiere, also Tiere, die nach Kaiserschnitt geboren wurden und steril gehalten wurden, sodass sich in ihrem Magen-Darm-Trakt keine natürliche Darmflora ausbilden konnte, mit krankheitserregenden Mikroorganismen infiziert und nach der Infektion entweder unbehandelt gelassen oder nach dem Auftreten der Krankheitssymptome wurden die erfindungsgemässen Produkte oral verabreicht. Es zeigte sich,dass nach einer Infektion mit krankheitserregenden Spezien der Familie Salmonella und auch krankheitserregenden Stämmen der Klasse Streptokokkus faecalis und krankheitserregenden Stämmen von Escherichia coli die Tiere der unbehandelten Gruppe schwer erkrankten und mehr als 50% der Tiere starben. Im Gegensatz dazu konnten die Krankheitssymptome nach der Verabreichung der erfindungsgemässen Präparate rasch gestoppt werden und die Ueberlebensrate betrug über 95%. Diese Tests wurden an unterschiedlichen gontobiotischen Tieren durchgeführt, beispielsweise Mäusen und Meerschweinchen.

Ein bevorzugter Stamm von Streptokokkus faecium, aus dessen Kulturmedium die erfindungsgemässen Produkte mit antibiotischer Wirksamkeit isolierbar sind, ist das Kulturmedium aus einer Züchtung des Stammes Streptokokkus faecium Cernelle 68 mit der Bezeichnung SF68.

Ein weiterer Gegenstand der vorliegenden Erfindung ist ein Verfahren zur Isolierung des erfindungsgemässen Produktes mit antibiotischer Wirksamkeit, wobei dieses Verfahren dadurch gekennzeichnet ist, dass man den Streptokokkus faecium in einem wässrigen Nährmedium züchtet bis in diesem Nährmedium einedas Wachstum von Stämmen von Streptokokkus faecalis und/oder Escherichia coli hemmende Wirksamkeit feststellbar ist, die Mikroorganismen aus dem wässrigen Nährmedium entfernt und verwirft und anschliessend das Produkt mit antimikrobieller Wirksamkeit aus dem wässrigen Nährmedium isoliert.

Gemäss einer bevorzugten Ausführungsart dieser Isolationsverfahren führt man zur Isolierung des Produktes mit antimikrobieller Wirksamkeit aus dem wässrigen Nährmedium in beliebiger Reihenfolge die folgenden Arbeitsschritte durch:

(a) Einstellung des pH-Wertes des wässrigen Mediums auf einen Wert im Bereich von 6,5 - 7,5 und Konzentrierung des wässrigen Mediums unter vermindertem Druck auf die Hälfte bis 1/20 des Ausgangsvolumens und

(b) Einstellung des pH-Wertes des Mediums auf einen Wert im Bereich von 6,0 - 9,0 und Extraktion des wässrigen Mediums mit einem wasserunmischbaren Lösungsmittel zur Entfernung von unpolaren wirkstoff-freien Bestandteilen aus dem wässrigen Medium.

Bevorzugte Lösungsmittel, die zur Entfernung von wenig polaren oder unpolaren Verunreinigungen aus dem einen pH-Wert im Bereich von 6,0 - 9,0 , insbesondere 6,0 - 7,5 , speziell bevorzugt 6,5 - 6,8 aufweisenden wässrigen Medium herangezogen werden können, sind aliphatische, alicyclische und aromatische Kohlenwasserstoffe, wie zum Beispiel entsprechende Erdölfraktionen, wie Petroläther, Benzol und Toluol, beziehungsweise halogenierte Kohlenwasserstoffe, insbesondere chlorierte Kohlenwasserstoffe, wie zum Beispiel Chloroform, Aethylenchlorid oder Tetrachlorkohlenstoff.

Es zeigte sich, dass bei dem Eindampfen der organischen Schicht, mit der die wenig polaren oder unpolaren Verunreinigungen aus dem etwa neutralen oder leicht alkalischen wässrigen Medium entfernt wurden, ein bräunlich gefärbter Rückstand verbleibt, der praktisch frei von antimikrobieller Wirksamkeit ist.

Bei diesem Reinigungsverfahren werden also die organischen Schichten verworfen.

Im allgemeinen wird bei der Durchführung der Isolation des Wirkstoffes, beziehungsweise Wirkstoffgemisches das von den Mikroorganismen abgetrennte wässrige Nährmedium zunächst unter vermindertem Druck eingeengt, im allgemeinen auf die Hälfte des Ausgangsvolumens bis auf 1/20 des Ausgangsvolumens und dann erst wird die Extraktion mit dem wasserunmischbaren organischen Lösungsmittel durchgeführt. Es ist jedoch auch möglich, zunächst das von den Mikroorganismen abgetrennte wässrige Nährmedium mit dem wasserunmischbaren organischen Lösungsmittel zu extrahieren um die oben genannten keine antimikrobielle Wirksamkeit aufweisenden Substanzen zu entfernen und anschliessend dann das wässrige Medium unter vermindertem Druck auf die Hälfte bis 1/20 des Ausgangsvolumens einzuengen.

Wie bereits erwähnt wurde, ist das Produkt mit antimikrobieller Wirksamkeit aus einem wässrigen Medium eines pH-Wertes von 3,0 oder unterhalb von 3,0 mit einem wasserunmischbaren organischen Lösungsmittel leicht extrahierbar. Gemäss einer bevorzugten Ausführungsart des erfindungsgemässen Verfahrens wird das Produkt mit antimikrobieller Wirksamkeit aus dem wässrigen Medium entfernt, indem man den pH-Wert des wässrigen Mediums durch Zugabe einer Säure auf einen Wert von 3,0 oder unter 3,0 einstellt, vorzugsweise durch Zugabe einer anorganischen Säure auf 1,5 - 2,5 , und bei diesem sauren pH-Wert den Wirkstoff durch Extraktion mit einem wasserunmischbaren Lösungsmittel aus der wässrigen Phase isoliert.

Im allgemeinen erfolgt diese Extraktion des erfindungsgemässen Produktes aus dem stark angesäuerten wässrigen Medium mit Hilfe des wasserunmischbaren organischen Lösungsmittels erst nach der Durchführung der Verfahrensschritte (a) und (b). Es ist jedoch auch möglich, im allgemeinen nach einer Konzentrierung des wässrigen Nährmediums unter vermindertem Druck, dieses durch Zugabe einer anorganischen Säure auf den stark sauren pH-Wert einzustellen, anschliessend dann mit dem mit Wasser nicht mischbaren organischen Lösungsmittel die Wirkstoffe mit antibiotischer Wirksamkeit zu extrahieren, anschliessend durch Zugabe von Ammoniak in die organische Phase übergegangene Säuren zu neutralisieren und das organische Lösungmittel abzudampfen und anschliessend dann den Rückstand in einem wässrigen oder wässrig-alkoholischen Lösungsmittel bei etwa neutralem pH-Wert aufzunehmen und aus dieser etwa neutralen oder allenfalls leicht basischen wässrigen Lösung die unpolaren Verunreinigungen durch Extraktion mit einem wasserunmischbaren Lösungsmittel zu entfernen.

Die zur Durchführung der Extraktion des Wirkstoffes aus dem einen stark sauren pH-Wert aufweisenden wässrigen Medium eingesetzten wasserunmischbaren organischen Lösungsmittel sind vorzugsweise etwas stärker polar als Kohlenwasserstoffe. Als bevorzugte Beispiele für verwendbare organische Lösungsmittel seien niedere aliphatische oder cyclische Aether, wie zum Beispiel Diäthyläther oder Ester niederer aliphatischer Säuren, wie der Essigsäure oder Propionsäure, wie zum Beispiel Essigsäureäthylester genannt.

Es zeigte sich, dass in dem wässrigen Kulturmedium von Streptokokkus faecium noch Produkte enthalten sind, die ein wesentlich höheres Molekulargewicht als 1000 Dalton aufweisen. Es ist zweckmässig, diese Verunreinigungen aus dem erfindungsgemässen Produkt zu entfernnen. Gemäss einer bevorzugten Arbeitsweise stellt man sicher, dass das wässrige Nährmedium, welches das erfindungsgemässe Produkt gelöst enthält, einen pH-Wert im Bereich von 6,0 - 7,5 aufweist und entfernt dann aus diesem wäss rigen Medium Nebenprodukte eines Molekulargewichts von 5000 oder höher, vorzugsweise 1000 oder höher, indem man eine Diffusion unter Verwendung einer Membran durchführt, welche die Substanzen des hohen Molekulargewichts nicht passieren lässt und/oder eine Filtration mit einem Ultramembranfilter mit einer Ausschlussgrenze von 5000, oder vorzugsweise einer Ausschlussgrenze von 1000, durchführt. Die erfindungsgemässen Wirkstoffe, welche ein Molekulargewicht von unter 1000 Dalton besitzen, passieren bei diesem Reinigungsschritt die Membran, beziehungsweise das Ultramembranfilter und diese antimikrobiellen Wirkstoffe werden dann aus dem diffundierten wässrigen Medium, beziehungsweise dem Ultrafiltrat isoliert.

Es ist auch möglich, eine Entfernung der hochmolekularen Bestandteile aus dem wässrigen Medium mit Hilfe einer Dialyse durchzuführen, bevor die Konzentrierung des von den Mikroorganismen abgetrennten wässrigen Nährmediums unter vermindertem Druck, beziehungsweise die Extraktion des etwa einen neutralen pH-Wert aufweisenden wässrigen Nährmediums mit dem wasserunmischbaren Lösungsmittel durchgeführt wird. Gemäss einer alternativen Ausführungsart erfolgt jedoch diese Entfernung der hochmolekularen Bestandteile durch Ultrafiltration und/oder Diffusion nach der Durchführung der Verfahrensschritte (a) und (b) und/oder nach der Isolierung des mindestens einen Wirkstoff enthaltenden Produktes aus dem einen sauren pH-Wert aufweisenden wässrigen Mediums durch Extraktion mit dem wasserunmischbaren organischen Lösungsmittel.

Es zeigte sich ferner, dass die Stoffe mit antimikrobieller Wirksamkeit aus dem wässrigen Medium von Aktivkohle im etwa neutralen pH-Bereich nicht absorbiert werden. Dementsprechend ist es auch möglich, unerwünschte Verunreinigungen aus dem wässrigen Medium mit Hilfe von Aktivkohle zu entfernen.

Die aus dem wässrigen Medium gewonnenen erfindungsgemässen Produkte mit antimikrobieller Wirksam keit können noch weiter gereinigt werden, indem man eine Säulenchromatographie durchführt. Als Füllung der Säule wird mit Vorteil ein Ionenaustauscher oder ein Absorptionsmittel verwendet, wie zum Beispiel Kieselgel.

Bei der Reinigung mit Hilfe der Chromatographie wird die Säule zunächst mit einem organischen Lösungsmittel oder Lösungsmittelgemisch entwickelt, das einen neutralen oder alkalischen pH-Wert aufweist. Die dabei austretenden Fraktionen sind vollständig oder im wesentlichen frei von Produkten mit antimikrobieller Wirksamkeit und sie werden dementsprechend verworfen.

Anschliessend erfolgt dann eine Elution der Chromatographiesäule mit einem organischen Lösungsmittel und/oder einem Lösungsmittelsystem, das einen sauren pH-Wert aufweist und bei Verwendung dieses Elutionsmittels treten dann aus der Säule Fraktionen aus, welche den Wirkstoff mit antimikrobieller Wirksamkeit, beziehungsweise die Wirkstoffe mit antimikrobieller Wirksamkeit, enthalten.

Zur Einstellung des sauren pH-Wertes des Elutionsmittels werden mit Vorteil niedrig molekulare organische Säuren eingesetzt, wie zum Beispiel Ameisensäure, Oxalsäure, Essigsäure oder Propionsäure.

Gemäss einer bevorzugten Ausführungsart des Verfahrens wird die chromatographische Reinigung des Produktes mit antimikrobieller Wirksamkeit durchgeführt, nachdem die Verfahrensschritte (a) und (b) des Reinigungsverfahrens bereits durchgeführt worden waren und nachdem das Produkt mit antimikrobieller Wirksamkeit aus dem einen sauren pH-Wert aufweisenden wässrigen Medium durch Extraktion mit dem wasserunmischbaren Lösungsmittel entfernt wurde.

Vorzugsweise führt man die chromatographische Reinigung des Produktes mit antimikrobieller Wirksamkeit unter Verwendung einer mit Ionenaustauscher oder einem Absorptionsmittel, z.B. Kieselsäure, gefüllten Chromatographiesäule durch, indem man die Chromatographiesäule zunächst mit einem organischen Lösungsmittel oder Lösungsmittelgemisch entwickelt, das einen neutralen oder alkalischen pH-Wert aufweist, die dabei austretenden Fraktionen, die keine oder nur eine ganz geringfügige antimikrobielle Wirksamkeit aufweisen, verwirft und anschliessend den mindestens einen antimikrobiellen Wirkstoff, beziehungsweise ein Produkt, das diesen enthält, aus der Chromatographiesäule eluiert, indem man mit einem organischen Lösungsmittel und/oder Lösungsmittelsystem eluiert, das einen sauren pH-Wert aufweist, beispielsweise einem Lösungsmittelgemisch, das eine organische Säure, beispielsweise Ameisensäure, Oxalsäure, Essigsäure oder Propionsäure enthält, vorzugsweise einem Lösungsmittelgemisch, das die entsprechende Säure, sowie einen niederen aliphatischen Alkohol, ein wasserunmischbares Lösungsmittel, beispielsweise Chloroform und ferner Wasser enthält, und dass man die unten austretenden Fraktionen, die eine antimikrobielle Wirksamkeit besitzen, konzentriert.

Auf die Chromatographiesäule wird vorzugsweise das aus der einen pH-Wert von 3,0 oder unter 3,0 aufweisenden wässrigen Phase mit einem organischen Lösungsmittel extrahierte Produkt aufgebracht, wobei dieses Produkt vorzugsweise vor dem Aufbringen mehrmals unter Vakuum abgedampft wurde, damit Spuren der anorganischen Säure, die aus dem wässrigen Medium mit dem organischen Lösungsmittel extrahiert worden waren, entfernt werden.

Anschliessend wird dann der Rückstand in einem basischen Lösungsmittelsystem aufgenommen und auf die Chromatographiesäule aufgebracht. Als Beispiel für ein zu diesem Zweck verwendbares alkalisches Lösungsmittelsystem sei eine Mischung aus Chloroform plus Methanol plus Wasser plus Ammoniak im Volumensverhältnis 90 : 54 : 5,5 : 5,5 genannt.

Dieser organische Extrakt wird dann auf eine Chromatographiesäule aufgebracht, vorzugs weise eine solche, die vorher mit dem gleich Lösungsmittelsystem äquilibriert wurde. Beispielsweise kann diese Chromatographiesäule als Füllung Kieselgel 60 enthalten. Zweckmässigerweise erfolgt die Elution der Chromatographiesäule zunächst ebenfalls mit dem oben genannten Ammoniak enthaltenden organischen Lösungsmittel und dann mit einem neutralen organischen Lösungsmittel, beispielsweise einer Mischung aus Chloroform plus Methanol plus Wasser im Volumenverhältnis von 90 : 54 : 11. Diese Elution mit dem neutralen Lösungsmittelsystem wird so lange durchgeführt, bis die austretenden Fraktionen einen neutralen pH-Wert aufweisen.

Alle Fraktionen, die mit dem alkalischen und dem neutralen Lösungsmittelsystem eluiert wurden, waren frei von antimikrobiellen Wirkstoffen und wurden verworfen. Anschliessend erfolgte dann die Elution mit Hilfe eines sauren Lösungsmittelsystems, beispielsweise einem System enthaltend Chloroform plus Methanol plus $H_2O$ plus Essigsäure im Volumenverhältnis von 50 : 25 : 4 : 8.

Bei dieser Elution mit dem sauren Lösungsmittelsystem traten dann Fraktionen aus der Chromatographiesäule aus, welche eine hohe antimikrobielle Wirkung aufwiesen. Einer der isolierten Wirkstoffe wies eine leicht gelbliche Farbe auf und er besass im Ultraviolettspektrum ein Absorptionsmaximum von 260 nm.

Des weiteren betrifft die Erfindung ein pharmazeutisches Präparat mit antimikrobieller Wirksamkeit, das dadurch gekennzeichnet ist, dass es als Wirkstoff das erfindungsgemässe Produkt enthält.

Die Erfindung wird nun anhand der Beispiele näher erläutert:

Beispiel 1

Züchtung des Mikroorganismenstammes Streptokokkus faecium Cernelle 68

Die Mikroorganismen wurden in einem Nährmedium gezüchtet, das pro Liter 45 g Glucose, 15 g Fleischpepton und 15 g Hefeextrakt enthielt.

Der pH-Wert dieses Nährmediums betrug 6,8 und die Züchtung des Mikroorganismenstammes Streptokokkus faecium Cernelle 68 erfolgte bei einer Temperatur von 35° während 12 Stunden. Nach dieser Zeit wurden die Mikroorganismen abzentrifugiert und verworfen.

Beispiel 2

Isolierung des Produktes aus dem wässrigen Medium

Ein Liter des nach dem Verfahren gemäss Beispiel 1 erhaltenen, von den Mikroorganismen befreiten Kulturmediums wurde unter Vakuum auf 1/10 seines Volumens eingeengt.

Der pH-Wert des eingeengten Kulturmediums wurde gemessen, falls er nicht im Bereich von 6,2 - 6,8 lag, durch Zugabe von Phosphatpuffer auf diesen pH-Bereich eingestellt.

Anschliessend wurde mit einem gleichen Volumen von Chloroform zweimal ausgeschüttelt und die organischen Phasen wurden verworfen.

Die wässrige Phase wurde anschliessend durch Zugabe von konzentrierter Chlorwasserstoffsäure auf einen pH-Wert von 2,0 gebracht. Sie wurde dreimal, jeweils mit einem etwa gleichen Volumen an Diäthyläther ausgeschüttelt. Die organischen Phasen wurden miteinander vereinigt und mehrmals unter Vakuum zur Trockene einge dampft. Dadurch wurden die letzten Spuren an in die Aetherphase übergegangener Chlorwasserstoffsäure entfernt. Man erhielt 12 g Trockenrückstand.

Der verbleibende Rückstand war das erfindungsgemässe Wirkstoffe enthaltende Produkt.

Beispiel 3

Isolierung des Produktes mit antimikrobieller Wirksamkeit aus dem wässrigen Medium

Zehn Liter des nach dem Verfahren gemäss Beispiel 1 gewonnen von den Mikroorganismen befreiten Zuchtmediums wurden unter Vakuum eingeengt, bis das Volumen 2,5 1 betrug.

Von diesem eingeengten Kulturmedium wurden jeweils Portionen zu 250 ml entnommen und jede dieser Portionen wurde gegen vier Liter destilliertes Wasser 48 Stunden lang dialysiert.

Bei dieser Dialyse trat der antimikrobielle Wirkstoff durch die Membran hindurch und befand sich schliesslich im Dialysat. Das Dialysat (4 Liter) wurde unter Vakuum auf ein Volumen von 250 ml konzentriert.

Der pH-Wert dieses Konzentrates wurde gemessen und falls er nicht im Bereich von 6,2 - 6,8 lag, durch Zugabe von Phosphatpuffer auf diesen pH-Bereich eingestellt.

Anschliessend wurde diese annähernd neutrale wässrige Phase zweimal mit je 250 ml Chloroform ausgeschüttelt und die organischen Phasen verworfen.

Nachdem die wässrige Phase durch Zugabe von Salzsäure auf einen pH-Wert von 2,0 gebracht worden war, wurde sie dreimal jeweils mit 350 ml Diäthyläther ausgeschüttelt.

Die wässrige Phase wurde sodann verworfen und die organischen Phasen miteinander vereinigt und mehr mals unter Vakuum zur Trockene eingedampft. Der verbleibende Rückstand war das erfindungsgemässen Wirkstoff enthaltende Produkt.

Bei diesem Reinigungsverfahren erhielt man aus einem Liter des von den Mikroorganismen befreiten Kulturmediums 10 g des Rückstandes, der eine hohe antimikrobielle Wirksamkeit besass.

Beispiel 4

Weitere Reinigung des antimikrobiell wirksamen Produktes

800 mg des trockenen Rückstandes, der nach dem Verfahren gemäss Beispiel 2 gewonnen wurde, beziehungsweise 800 mg des trockenen Rückstandes, der nach dem Verfahren gemäss Beispiel 3 gewonnen wurde, wurden diesem weiteren Reinigungsverfahren unterworfen.

Dieses Reinigungsverfahren wurde durchgeführt, indem man zwei Chromatographiesäulen zur Durchführung der chromatographischen Trennung verwendete.

Die 800 mg des gemäss Beispiel 2, beziehungsweise Beispiel 3, gewonnenen Rückstandes wurden in 40 ml einer Mischung aus Chloroform plus Methanol plus Wasser plus Ammoniak im Mischungsverhältnis 90 : 54 : 5,5 : 5,5 gelöst.

Diese organischen Extrakte wurden jeweils auf eine 2,5 x 40 cm Säule aufgetragen, die mit Kieselgel 60 (fein) gefüllt und mit der oben angegebenen basischen Lösungsmittelmischung äquilibriert war.

Jede der beiden Säulen wurden zunächst mit der gleichen basischen Lösungsmittelmischung eluiert und dann mit einem Gemisch aus Chloroform plus Methanol plus Wasser im Mischungsverhältnis 90 : 54 : 11 weiter eluiert, bis schliesslich die aus den Säulen austretenden Fraktionen einen neutralen pH-Wert erreicht hatten.

Alle diese Fraktionen waren wirkstofffrei und wurden verworfen.

Anschliessend erfolgte dann eine Elution der beiden Säulen unter sauren Bedingungen, unter Verwendung eines Lösungsmittelgemisches aus Chloroform plus Methanol plus Wasser plus Essigsäure im Volumenverhältnis 50 : 25 : 4 : 8.

Bei dieser Elution mit dem sauren Elutionsmedium traten aus den beiden Säulen je drei Fraktionen aus, von denen die ersten beiden nach dem Eindampfen leicht gelblich waren, während die dritte Fraktion dunkelorange gefärbt war.

Die drei Fraktionen, die aus jeder der beiden Säulen bei der Elution mit dem sauren Medium aufgefangen worden waren, hatten eine sehr hohe antimikrobielle Aktivität.

Die Fraktionen 1 und 2 derjenigen Säule, auf der das Produkt gemäss Beispiel 3 chromatographiert worden war, wurden dann miteinander vereinigt und auf eine 10 ccm DE-52 Säule aufgetragen, die mit 0,05 molarer Ammoniumbikarbonatlösung eines pH-Wertes von 8,1 äquilibriert war. Die auf diese Säule aufgetragene Trockenmenge der Fraktionen 1 und 2 betrug 8 mg und die Säule wurde mit dem zur Aequilibrierung verwendeten Lösungsmittel entwickelt, wobei 50 - 55 % der Gesamtmenge des auf die Säule aufgetragenen Produktes in den austretenden Fraktionen gewonnen wurden. Diese Fraktionen waren biologisch aktiv.

Die Fraktionen wurden vom Ammoniumsalz befreit und das biologisch aktive Material zeigte ein Absorptionsmaximum bei 260 nm.

Schliesslich wurde die Säule mit einer 0,5 molaren Losung von Ammoniumbikarbonat eluiert, die austretenden Fraktionen wiesen jedoch keine antimikro bielle Wirksamkeit auf.

Beispiel 6

Weitere Reinigung des nach dem Verfahren gemäss Beispiel 2, bzw. Beispiel 3 gewonnenen Trok- kenrückstandes

Kleine Portionen diese Trockenrückstandes wurden auf chromatographische Säulen aufgetragen, die entweder Sephadex G-10 oder Sephadex AG1-X2 enthielten.

Auch bei diesen Reinigungsverfahren mit Hilfe der genannten Säulen traten bei der Elution mit wässrigen neutralen Lösungsmittelsystemen aus den Säulen keine wirkstoffhaltigen Fraktionen aus.

Anschliessend traten dann bei der Verwendung von 0,5-molarer wässriger Ameisensäure die gelblichen wirkstoffenthaltenden Fraktionen aus den verwendeten Chromatographiesäulen aus.

Nach der Abdampfung des Lösungsmittels unter Vakuum blieben Wirkstoff-Fraktioen mit hoher antimikrobieller Wirksamkeit zurück.

**Ansprüche**

1. Produkt mit antimikrobieller Wirksamkeit, dadurch gekennzeichnet, dass es aus dem Kulturmedium von Streptokokkus faecium isoliert ist und dass dieses Produkt mindestens einen Wirkstoff enthält oder aus mindestens einem Wirkstoff besteht, der ein Molekulargewicht unter 1000 Dalton aufweist und die folgenden Eigenschaften besitzt:

(a) der mindestens eine Wirkstoff verliert bei einem pH-Wert im Bereich von 6,2 - 7,5 bei einer Behandlung mit einem Peptidbindungen spaltenden Enzym seine Wirksamkeit nicht;
(b) der mindestens eine Wirkstoff verliert bei einer dreissigminütigen Erhitzung auf 60$^\circ$ und/oder zwanzigminütigen Erhitzung auf 80$^\circ$ seine Wirksamkeit nicht und
(c) der mindestens eine Wirkstoff ist in einem wässrigen Medium eines etwa neutralen bis alkalischen pH-Wertes im Bereich von 6,0 - 9,0 löslich und er ist aus dem wässrigen Medium dieses etwa neutralen bis schwach alkalischen pH-Wertes mit einem wasserunmischbaren organischen Lösungsmittel nicht extrahierbar.

2. Produkt gemäss Anspruch 1, dadurch gekennzeichnet, dass der mindestens eine Wirkstoff aus einem wässrigen sauren Medium eines pH-Wertes von 3,0 oder unterhalb von 3,0 , insbesondere eines pH-Wertes im Bereich von 1,5 - 2,5 mit einem wasserunmischbaren organischen Lösungsmittel extrahierbar ist.

3. Produkt gemäss Patentanspruch 1 oder 2, dadurch gekennzeichnet, dass der mindestens eine Wirkstoff eine wachstumshemmende Wirksamkeit gegenüber mehreren grampositiven und gramnegativen Bakterien aufweist, beispielsweise einem oder mehreren Stämmen von Streptokokkus faecalis, Stämmen von Escherichia coli, Salmonella, Staphylococcus aureus, Shigella, Bacillus subtilis und/oder Listeria.

4. Produkt gemäss einem der Patentansprüche 1 - 3, dadurch gekennzeichnet, dass das mindestens einen Wirkstoff enthaltende Produkt aus dem Kulturmedium aus einer Züchtung des Stammes Streptokokkus faecium Cernelle 68 mit der Bezeichnung SF68 isolierbar ist.

5. Produkt gemäss einem der Patentansprüche 1 - 4, dadurch gekennzeichnet, dass das mindestens einen Wirkstoff enthaltende Produkt in einem wässrigen Medium bei einem pH-Wert von 6,2 - 7,5 seine Wirksamkeit bei Anwesenheit von solchen Peptidbindungen spaltenden Enzymen nicht verliert, die aus der Gruppe ausgewählt sind, welche Desoxyribonuklease, Ribonuklease, Trypsin und Pronase umfasst.

6. Verfahren zur Isolierung des Produktes mit antimikrobieller Wirksamkeit gemäss Anspruch 1, dadurch gekennzeichnet, dass man den Streptokokkus faecium in einem wässrigen Nährmedium züchtet bis in diesem Nährmedium eine das wachstum von Stämmen von Streptokokkus faecalis und/oder Escherichia coli hemmende Wirksamkeit feststellbar ist, die Mikroorganismen aus dem wässrigen Nährmedium entfernt und verwirft und anschliessend das Produkt mit antimikrobieller Wirksamkeit aus dem wässrigen Nährmedium isoliert.

7. Verfahren nach Anspruch 6, dadurch gekennzeichnet, dass man zur Isolierung des Produktes mit antimikrobieller Wirksamkeit aus dem wässrigen Nährmedium in beliebiger Reihenfolge die folgenden Arbeitsschritte durchführt:

(a) Einstellung des pH-Wertes des wässrigen Mediums auf einen Wert im Bereich von 6,5-7,5 und Kon zentrierung des wässrigen Mediums unter vermindertem Druck auf die Hälfte bis 1/20 des Ausgangsvolumens und
(b) Einstellung des pH-Wertes des Mediums auf einen Wert im Bereich von 6,0 - 9,0 und Extraktion des wässrigen Mediums mit einem wasserunmischbaren Lösungsmittel zur Entfernung von unpolaren wirkstofffreien Bestandteilen aus dem wässrigen Medium.

8. Verfahren nach Anspruch 6 oder 7, dadurch gekennzeichnet, dass man das Produkt mit antimikrobieller Wirksamkeit aus dem wässrigen Medium entfernt, indem man den pH-Wert des wässrigen Mediums durch Zugabe einer Säure auf einen Wert von 3,0 oder unter 3,0 einstellt, vorzugsweise durch Zugabe einer anorganischen Säure auf 1,5 - 2,5 , und bei diesem sauren pH-Wert den Wirkstoff durch Extraktion mit einem wasserunmischbaren Lösungsmittel aus der wässrigen Phase entfernt.

9. Verfahren nach einem der Patentansprüche 6 - 8, dadurch gekennzeichnet, dass man aus dem wässrigen, einen pH-Wert von 6,0 - 7,5 aufweisenden Medium Produkte eines Molekulargewichts von 5000 oder höher entfernt, indem man eine Diffusion unter Verwendung einer Membran, welche die Substanzen des hohen Molekulargewichts nicht passieren lässt, durchführt und/oder eine Filtration mit Hilfe eines Ultramembranfilters durchführt, wobei das Produkt mit der antimikrobiellen Wirksamkeit in dem diffundierten wässrigen Medium, beziehungsweise dem Ultrafiltrat enthalten ist.

10. Verfahren gemäss einem der Patentansprüche 6 - 9, dadurch gekennzeichnet,dass man eine Reinigung des Produktes mit antimikrobieller Wirksamkeit unter Verwendung einer mit Ionenaustauscher oder einem Absorptionsmittel, z.B. Kieselsäure, gefüllten Chromatographiesäule durchführt, wobei die Chromatographiesäule zunächst mit einem organischen Lösungsmittel oder Lösungsmittelgemisch entwickelt wird, das einen neutralen oder alkalischen pH-Wert aufweist, die dabei austretenden Fraktionen, die keine oder nur eine ganz geringfügige antimikrobielle Wirksamkeit aufweisen, verwirft und anschliessend den mindestens einen antimikrobiellen Wirkstoff, beziehungsweise ein Produkt, das diesen enthält, aus der Chromatographiesäule eluiert, indem man mit einem organischen Lösungsmittel und/oder Lösungsmittelsystem eluiert, das einen sauren pH-Wert aufweist, beispielsweise einem Lösungsmittelgemisch, das eine organische Säure, beispielsweise Ameisensäure, Oxalsäure, Essigsäure oder Propionsäure enthält, vorzugsweise einem Lösungsmittelgemisch, das die entsprechende Säure, sowie einen niederen aliphatischen Alkohol, ein wasserunmischbares Lösungsmittel, beispielsweise Chloroform und ferner Wasser enthält, und dass man die unten austretenden Fraktionen, die eine antimikrobielle Wirksamkeit besitzen, konzentriert.

11. Verfahren nach einem der Patentansprüche 6 - 10, dadurch gekennzeichnet, dass man mindestens zwei Fraktionen mit antimikrobieller Wirksamkeit isoliert, wobei die eine dieser wirksamen Fraktionen leicht gelblich ist und im Ultraviolettspektrum ein Absorptionsmaximum bei 260 nm aufweist.

12. Pharmazeutisches Präparat mit antimikrobieller Wirksamkeit, dadurch gekennzeichnet, dass es als Wirkstoff das Produkt gemäss Anspruch 1 enthält.

13. Pharmazeutisches Präparat gemäss Anspruch 12, dadurch gekennzeichnet, dass es als Wirkstoff ein Produkt gemäss einem der Ansprüche 2 - 5 enthält.

14. Pharmazeutisches Präparat gemäss Anspruch 12 oder 13, dadurch gekennzeichnet, dass es ein oral zu verabreichendes Produkt zur Bekämpfung von Störungen im Magen-Darm-Trakt oder zur prophylaktischen Vorbeugung gegen Störungen im Magen-Darm-Trakt ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | CHEMICAL ABSTRACTS, Band 107, 1987, Seite 382, Zusammenfassung Nr. 4130t, Columbus, Ohio, US; M.J. KIM et al.: "Identification of Streptococcus faecalis and Streptococcus faecium and susceptibility studies with newly developed antimicrobial agents", & J. CLIN. MICROBIOL. 1987, 25(5), 787-90 * Zusammenfassung * --- | 1,3,6, 12 | C 12 P 1/04 A 61 K 35/66 // (C 12 P 1/04 C 12 R 1:46 ) |
| Y | CHEMICAL ABSTRACTS, Band 105, 1986, Seite 353, Zusammenfassung Nr. 11878e, Columbus, Ohio, US; & JP-A-61 53 293 (ADVANCE KAIHATSU KENKYUSHO K.K.) 17-03-1986 * Zusammenfassung * --- | 1,3,6, 12 | |
| Y | CHEMICAL ABSTRACTS, Band 105, 1986, Seite 568, Zusammenfassung Nr. 151550s, Columbus, Ohio, US; & JP-A-61 109 728 (ADVANCE KAIHATSU KENYUSHO K.K.) 28-05-1986 * Zusammenfassung * --- | 1,3,6, 12 | |
| Y | CHEMICAL ABSTRACTS, Band 91, 1979, Seiten 61-62, Zusammenfassung Nr. 186755q, Columbus, Ohio, US; R.C. MOELLERING et al.: "Species-specific resistance to antimicrobial synergism in Streptococcus faecium and Streptococcus faecalis", & J. INFECT. DIS. 1979, 140(2), 203-8 * Zusammenfassung * --- | 1,3,6, 12 | **RECHERCHIERTE SACHGEBIETE (Int. Cl.5)** C 12 P C 12 R A 61 K |
| A | US-A-4 210 673 (KVANTA) * Insgesamt * --- -/- | 1,6,12 | |

Der vorliegende Recherchenbericht wurde für alle Patentansprüche erstellt

| Recherchenort | Abschlußdatum der Recherche | Prüfer |
|---|---|---|
| DEN HAAG | 04-10-1990 | RAJIC M. |

Europäisches
Patentamt

Nummer der Anmeldung

EP  90 11 2401

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A-2 550 223  (S. UDAKA) <br> * Insgesamt * <br> --- | 1,6,12 | |
| A | ANTIMICROBIAL AGENTS AND CHEMOTHERAPY, Band 32, Nr. 1, Januar 1988, Seiten 24-26, American Society for Microbiology; L.D. LIEBOWITZ et al.: "In vitro selection of bacteria resistant to LY146032, a new cyclic lipopeptide" <br> * Insgesamt * <br> ----- | 1,6,12 | |

RECHERCHIERTE
SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| DEN HAAG | 04-10-1990 | RAJIC M. |

EPO FORM 1503 03.82 (P0403)